Europäisches Patentamt

European Patent Office

Office européen des brevets

(11) Veröffentlichungsnummer: **0 507 278 A2**

## (12) EUROPÄISCHE PATENTANMELDUNG

(21) Anmeldenummer: **92105616.4**

(22) Anmeldetag: **01.04.92**

(51) Int. Cl.⁵: **C12N 11/14**, C12P 7/62,
C12P 41/00

(30) Priorität: **02.04.91 DE 4110637**

(43) Veröffentlichungstag der Anmeldung:
**07.10.92 Patentblatt 92/41**

(84) Benannte Vertragsstaaten:
**AT BE CH DE DK FR GB IT LI NL SE**

(71) Anmelder: **HOECHST AKTIENGESELLSCHAFT
Postfach 80 03 20
W-6230 Frankfurt am Main 80(DE)**

(72) Erfinder: **Schneider, Manfred, Prof. Dr.
Triebelsheider Weg 47
W-5600 Wuppertal(DE)**
Erfinder: **Laumen, Kurt, Dr.
Steinackerweg 10
W-7806 March 2(DE)**
Erfinder: **Fülling, Gerd, Dr.
Drosselweg 3
W-6230 Frankfurt am Main(DE)**
Erfinder: **Holla, Wolfgang, Dr.
Hasenpfad 1-3
W-6239 Kriftel(DE)**
Erfinder: **Keller, Reinhold, Dr.
Alleestrasse 18
W-6232 Bad Soden am Taunus(DE)**
Erfinder: **Kretzschmar, Gerhard, Dr.
Ulmenweg 10
W-6236 Eschborn(DE)**
Erfinder: **Schudok, Manfred, Dr.
Birminghamstrasse 89
W-6230 Frankfurt am Main(DE)**
Erfinder: **Wullbrandt, Dieter, Dr.
Zum Lindenhof 11
W-6238 Hofheim am Taunus 5(DE)**

(54) Immobilisierter Biokatalysator, dessen Herstellung und Verwendung zur Estersynthese in einem Säulenreakfor.

㊼ Die Erfindung betrifft ein Verfahren zur Acylierung von Alkoholen, mit Ausnahme der tertiären Alkohole, mittels Lipasen aus Pseudomonas, Mucor, Candida, Rhizopus, Penicillium oder Schweinepankreas, die an inerten Trägern (Seesand, Gesteinsmehl, Ziegelmehl, Glas, pulverisierte Keramiken) immobilisiert sind.

EP 0 507 278 A2

Die Synthese von Estern aus Carbonsäuren oder Carbonsäurederivaten und Alkoholen ist eine zentrale Aufgabenstellung im Rahmen der Organischen Chemie. Beispielsweise finden Ester Anwendung als Fette, Öle, Emulgatoren, Geruchsstoffe, Pharmaka, Pflanzenschutzmittel, Flüssigkristall-Bausteine etc. Im allgemeinen erfolgt die Estersynthese in Gegenwart eines Katalysators. Es ist bekannt, daß die Katalysatorfunktion von Enzymen ausgeübt werden kann.

Viele der oben erwähnten Stoffe sind optisch aktiv. Zu deren Synthese werden oft einfach enantiomerenreine Ausgansprodukte benötigt. Diese sind z.T. aus optisch reinen Naturstoffen zu gewinnen, häufig muß jedoch der Weg der enantiodifferenzierenden Synthese, ausgehend von racemischen oder prochiralen Verbindungen, beschritten werden. Da insbesondere optisch aktive Alkohole, Carbonsäuren und Carbonsäureester eine große wirtschaftliche Bedeutung besitzen, kommt deshalb einem wirtschaftlichen Herstellungsverfahren zur enantiodifferenzierenden Acylierung von Alkoholen bzw. mit Carbonsäuren eine große Bedeutung zu. Gleiches gilt für die stereo- und/oder regioselektive Acylierung von Mono-, Di- oder Polyolen (z.B. Kohlenhydraten).

Enzyme bieten darüberhinaus den Vorteil, daß neben unverzweigten Alkoholen bzw. Carbonsäuren auch racemische oder prochirale Alkohole bzw. Carbonsäuren regio-, enantio- und diastereoselektiv unter schonendsten Bedingungen acyliert werden können, so daß beispielsweise die Acylierung von Alkoholen im Sinne einer Racematspaltung verläuft oder daß prochirale Alkohole asymmetrisch acyliert werden.

Es ist bereits bekannt, daß man Vinylester unter Hinzugabe von Alkoholen in Gegenwart von Lösungsmitteln, wie z.B. Tetrahydrofuran, unter enzymatischer Katalyse umestern kann (M. Degueil-Castaing et al., Tetrahedron Letters, Vol. 28, Nr. 9, Seiten 953-954, 1987). Als Enzym wurde Schweinepankreas-Lipase benutzt. Eine Stereoselektivität wurde nicht beobachtet.

Es ist außerdem die enzymatische Trennung von racemischen Alkoholen aufgrund einer selektiven, enzymkatalysierten Umesterungsreaktion mit Vinylestern in Abwesenheit von Lösungsmitteln bekannt. Als Enzyme dienen immobilisierte Lipasen aus Schweineleber und -pankreas sowie aus den Mirkoorganismen Pseudomonas, Candida, Mucor, Rhizopus und Penicillium (EP 0 321 918).

Kontinuierliche enzymatische Verfahren können besonders vorteilhaft eingesetzt werden. Dazu werden die Enzyme im allgemeinen in immobilisierter Form eingesetzt. Dies bietet den Vorteil einer breiten Anwendbarkeit und einer nahezu beliebigen Maßstabsvergrößerung des Verfahrens. Voraussetzung dafür ist ein wirtschaftlicher Zugang zu geeigneten Trägermaterialien, sowie ein einfaches, schnelles und kostengünstig durchführbares Immobilisierungsverfahren.

So ist außerdem beispielsweise bekannt, daß Lipasen zur Hydrolyse und Umesterung von Fetten, Ölen und ähnlichen Verbindungen immobilisiert an anionische lonenaustauscher eingesetzt werden können [(M. Mittelbach, J. Am. Oil Chemist's Society, 67, 168-170 (90)].

Meistens werden für die Immobilisierung von Enzymen bestimmte Standard-Verfahren eingesetzt. Das Enzym wird adsorptiv, ionisch oder kovalent an einen geeigneten Träger fixiert (I. Chibata, Immobilized Enzyms: Research and Development, Halsted Press, Wiley, New York, 1978 S.5-7; W Hartmeier: Immobilisierte Biokatalysatoren, Springer Verlag Berlin Heidelberg, New York, Tokyo 1986 S.14-16).

Es wurde nun gefunden, daß sowohl die Immobilisierung von Hydrolasen an inerten, anorganischen Trägern, als auch die Anwendung der Immobilisate zur Estersynthese im organischen Reaktionsmilieu wesentlich einfacher und wirtschaftlicher als bei bekannten Verfahren durchgeführt werden kann, indem der inerte, anorganische Träger und die Hydrolase in einfacher Form miteinander gemischt werden. Als inerte Träger werden solche bezeichnet, die keine aktive Oberfläche besitzen, welche bei herkömmlichen Verfahren für die Enzym-Träger-Beziehung (ionische oder kovalente Bindung) notwendig ist. Dieses einfache Verfahren ist bislang noch nicht beschrieben worden und bietet den Vorteil, daß das Katalysezentrum des Enzyms nicht durch Bindungsbildung blockiert ist.

Die Erfindung betrifft somit:

1. Einen immobilisierten Biokatalysator, aus einem inerten, anorganischen Träger, und einer Hydrolase, die an diesen Träger immobilisiert ist.

2. Einen Biokatalysatorträger nach Anspruch 1, aus den Materialien Seesand, Gesteins-, Ziegelmehl, Glas oder pulverisierter Keramik.

3. Ein Verfahren zur Herstellung eines immobilisierten Biokatalysators, das dadurch gekennzeichnet ist, daß die Hydrolase mit einem inerten, anorganischen Träger aus Seesand, Gesteinsmehl, Ziegelmehl, Glas oder pulverisierter Keramik gemischt und daran immobilisiert ist.

4. Verwendung des Verfahrens zur Estersynthese in einem Säulenreaktor.

Im folgenden wird die Erfindung im einzelnen beschrieben. Ferner wird die Erfindung durch den Inhalt der Ansprüche bestimmt.

Das Verfahren ist anwendbar auf alle Umsetzungen, d.h. Acyltransfer-Reaktionen, die in organischen Lösemitteln durch Hydrolasen katalysiert werden. Die möglichen Reaktionen sind entweder aus der Literatur bekannt oder sind durch Vorversuche leicht zu bestimmen.

Als Trägermaterialien für die Enzymfixierung dienen inerte, anorganische Materialien wie Gesteinsmehl, Ziegelmehl, pulverisierte Keramiken, vorzugsweise jedoch Seesand.

Alle Materialien sind käuflich erhältlich oder sehr einfach zu gewinnen und müssen nicht vor- oder nachbehandelt werden. Vorzugsweise werden die Träger jedoch noch zerkleinert und klassiert (Standardverfahren). Seesand ist im geglühten Zustand käuflich (Riedel-de Haen).

Als Enzyme, welche die Veresterung katalysieren, werden Hydrolasen, insbesondere Lipasen aus Mucor, Rhizopus, Penicillium, Pseudomonas oder die Schweinepankreas-Lipase verwendet, vorzugsweise jedoch die Lipase aus Pseudomonas (Lipase P, auch als FP oder PS bezeichnet, Amano Pharmaceuticals, Nagoja, Japan) oder aus Candida (z.B. Sigma Chemicals Co., St. Louis, MO, USA bzw. Lipase OF (Meito Sangyo, Nagoja, Japan).

Für die Immobilisierung des Enzyms werden Hydrolase und Träger vermischt, vorzugsweise in trockenem Zustand.

Die Korngröße des Trägers beträgt maximal 2 mm, vorzugsweise 0,01 - 2 mm, insbesondere jedoch 0,1 - 1 mm, muß jedoch für die Durchführung des erfindungsgemäßen Verfahrens nicht einheitlich sein.

Die mit Enzym zu belegende Trägermenge wird in Abhängigkeit von der Größe des Ansatzes, von der zu erwartenden Reaktionszeit und von der Umsatzhöhe gewählt. Sie kann durch Vorversuche leicht bestimmt werden.

Die immobilisierte Hydrolase ist dann unter trocknen Lagerbedingungen bei Raumtemperatur mit voller Enzymaktivität überraschend lange stabil. Die Halbwertszeit des immobilisierten Enzyms bei Benutzung im Labor beträgt ≧ 200 Tage.

Bei der Acyl-Transferreaktion wird die als Lösemittel dienende oder in einem anderen organischen Lösemittel gelöste Acylkomponente in ein Keton, Aldehyd, Alkohol oder Wasser und einen Acyl-Enzym-Komplex gespalten, wobei letzterer mit dem hinzugegebenen Alkohol (Substrat) reagiert.

Als Acyldonoren werden verzweigte und unverzweigte Carbonsäuren, Carbonsäureester, Carbonsäureanhydride, vorzugsweise jedoch Vinylester eingesetzt. Die Reaktion verläuft im Sinne eines Acyltransfers. Sind im Acyldonor oder im Alkohol stereogene Zentren enthalten, verläuft die Umsetzung als enantio-, regio- oder diastereodifferenzierender Acyltransfer.

Die Vinylester sind nach dem Fachmann bekannten Verfahren einfach herzustellen oder käuflich erhältlich.

Gleiches gilt für die Carbonsäureester, die Carbonsäuren und die cyclischen Carbonsäureanhydride.

Als zu acylierende Alkohole können verzweigte und unverzweigte Alkohole, mit Ausnahme der tertiären Alkohole, eingesetzt werden. Die Alkohole erhält man, sofern sie nicht käuflich sind, z.B. durch Reduktion aus den entsprechenden Ketonen oder Carbonsäureestern, die meist käuflich sind, oder durch $\alpha$-Halogenie-rung entsprechender Ketone mit anschließender Reduktion zum Alkohol. Andere nicht käufliche Verbindungen lassen sich nach literaturbekannten Verfahren einfach herstellen, beispielsweise über Grignard- oder andere gängige Additionsreaktionen.

Für die Acylierungsreaktion werden vorzugsweise eingesetzt:

1-Phenylethanol, 1-Phenylpropanol, 2-Chlor-1-phenylethanol, ( + -)-Dihydro-4,4-dimethyl-3-hydroxy-2-(3H)-furanon (Pantolacton), 2-Hydroxypropanaldimethylacetal, 4-Hydroxy-3-methyl-2-(prop-2-enyl)-cyclopent-2-enon (Allethrolon), [2-(6-Acetoxy)naphthyl]-1-ethanol, 1-Octen-3-ol, 2-(1-Naphthylmethyl)-propan-1,3-diol, 1-(4-Isobutylphenyl)ethanol, 1,5-Anhydro-D-arabino-hex-1-enitol (Glucal).

Der zu acylierende Alkohol oder die zu veresternde Carbonsäure werden in Konzentrationen von 0,05 - 200 %, bezogen auf das Volumen der einzusetzenden Acylkomponenten bzw. deren Lösung in einem inerten organischen Lösemittel, eingesetzt. Ein weiter Konzentrationsbereich ist möglich und hängt von der Reaktivität des umzusetzenden Substrats ab.

Die Acylierung wird im diskontinuierlichen oder kontinuierlichen Säulenbetrieb durchgeführt. Das Prinzip des Säulenbetriebs ist ein Standardverfahren.

Für den Säulenlauf wird eine temperierbare Doppelmantel-Glassäule mit Fritte zunächst mit Träger, dann mit dem Enzym-Träger-Gemisch und zuletzt mit Träger gefüllt.

Enzym und Träger werden im Verhältnis 1:100 bis 1:2, bevorzugt 1:20 - 1:5 (Gew./Gew.) eingesetzt.

Die Säulendimension ist wählbar, abhängig von der Reaktivität des Substrats und der umzusetzenden Substratmenge. Aufgrund der einfachen Herstellungsweise des immobilisierten Biokatalysators durch Mischen von Enzym und inertem, anorganischem Material eignet sich das Verfahren besonders für industrielle Anwendungen.

Die Temperatur der Säule beträgt -10 bis +80°C, vorzugsweise 10 - 40°C und ist besonders von der zu erwartenden Reaktionsgeschwindigkeit, der benötigten Selektivität und der Stabilität des Enzyms abhängig.

Für die enzymatische Acylierung im diskontinuierlichen Säulenbetrieb wird der Alkohol oder die Carbonsäure in dem Acyldonor oder Alkohol bzw. in einer Lösung des Acyldonors oder des Alkohols aufgenommen und das Gemisch mittels einer Pumpe kontinuierlich umgepumpt.

Der diskontinuierliche Säulenbetrieb bietet den Vorteil einer leicht durchführbaren Umsatzkontrolle und auch die Möglichkeit, die Umsatzhöhe jederzeit einzustellen.

Im kontinuierlichen Säulenbetrieb passiert die entsprechende Lösung mit oder ohne Pumpanlage die mit immobilisiertem Biokatalysator gefüllte Säule.

Die Höhe des Umsatzes im kontinuierlichen Verfahren kann nahezu beliebig durch Einstellen der Tropfgeschwindigkeit gewählt werden und muß durch Vorversuche vorher bestimmt werden.

Die Raum-Zeit-Ausbeuten sind in beiden Verfahren direkt von den Absolutwerten der oben genannten Parameter abhängig, besonders aber von der immobilisierten Enzymmenge.

Die im diskontinuierlichen oder kontinuierlichen Verfahren erhaltene Produktlösung wird anschließend destillativ vom Lösemittel befreit.

Die Reinheitskontrolle bzw. Umsatzbestimmung erfolgt mit üblichen analytischen Methoden, z.B. DC, GC, $^1$H-NMR-Spektroskopie und durch Drehwertmessungen.

In den nachfolgenden Beispielen wird die Erfindung detailliert erläutert.

1) Herstellung des immobilisierten Biokatalysators

Der inerte, anorganische Träger wird nach Zerkleinern, Glühen und Kassieren bzw. direkt (Seesand) eingesetzt.

Man stellt durch Vermischen von 5 g Lipase P mit 50 ml käuflich geglühtem Seesand eine homogene Mischung her.

Die temperierbare Doppelmantel-Glassäule wird dann mit 25 ml Seesand gefüllt, 55 ml des Enzym-Seesand-Gemischs auf den Seesand geschichtet und nochmals mit 20 ml Seesand überschichtet.

2) Anwendung zur Acylierung im diskontinuierlichen Betrieb

30 g racemisches 4-Hydroxy-3-methyl-2-(prop-2-enyl)-cyclopent-2-enon (Allethrolon) werden in 180 ml Vinylacetat aufgenommen und für 23 Std. kontinuierlich umgepumpt (Fördervolumen: 6 - 10 l/h). Das Vorratsgefäß mit dem Substrat wird ebenfalls während des gesamten Verfahrens temperiert, so daß die Temperaturen in Säule und Vorratsgefäß identisch sind.

Nach Ablauf der Reaktionszeit (= Dauer des Umpumpens: 23 h) wird die Substratlösung abgepumpt und die Säule dreimal mit je 50 ml Vinylacetat nachgewaschen.

Zur quantitativen Entfernung des Lösungsmittels wird die Substratlösung zunächst für 2 h bei 400 mbar und 60°C und dann nochmals für 2 h bei 50 mbar und 60°C destilliert.

| Umsatzbestimmungen mittels GC: | 35 % Acetat |
|---|---|
| | 65 % Alkohol |

Ausbeute: (Bestimmung der Enantiomerüberschüsse mittels $^1$H-HMR-Shift-Versuche mit Eu(hfc)$_3$ sowie Drehwertmessungen)

Acetat: ee ≥ 95 %
Drehwert: -31,4 (1-2 %ige Lösung in Chloroform)

Alkohol: ee ≥ 40 %
Drehtwert: +5,3 (1-2 %ige Lösung in Chloroform)

| Drehwert der reinen Verbindungen: | |
|---|---|
| Acetat: | -32,3 |
| Alkohol: | -13,9 |

Anschließend wird die Reaktion noch 159 x in gleicher Weise durchgeführt. Der Umsatz sinkt dabei auf 18 %.

3) Anwendung zur Acylierung im kontinuierlichen Säulenbetrieb

Eine Lösung von 1,2 kg (6,74 mol) 1-(4-Isobutylphenyl)-ethanol in 1,2 l Vinylacetat wird mit einer Tropfgeschwindigkeit von 16 ml/h über eine Biokatalysatorsäule gegeben. Die Säule (Durchmesser: 8 cm; Länge: 40 cm) wurde mit 150 ml Seesand gefüllt, darüber eine Mischung aus 250 ml Seesand und 50 g Lipase P gegeben und anschließend mit 100 ml Seesand überschichtet. Überschüssiges Vinylacetat in der Produktlösung wird am Rotationsverdampfer entfernt und das Produktgemisch durch $^1$H-NMR auf

Umsatz untersucht.

Umsatz: 50 %

Das Produktgemisch wird durch Säulenchromatographie an Kieselgel (Laufmittel Hexan/Essigester, 50:1 Vol/Vol) aufgetrennt.

| Ausbeute: | |
| --- | --- |
| 650 g Acetat | 44 % |
| 580 g Alkohol | 48 % |

Acetat:  Drehwert $[\alpha]_D^{20}$ = +97,6 (c = 1, CHCl$_3$)
klare Flüssigkeit
ee $\geq$ 95 % ($^1$H-NMR + Shift)

Alkohol:  Drehwert $[\alpha]_D^{20}$ = -41,6 (c 1, CHCl$_3$)
Schmp.: 33 °C
ee $\geq$ 95% ($^1$H-NMR + Shift)

4) Regioselektive Diacetylierung von D-Glucal (1,5-Anhydro-D-arabino-hex-1-enitol) zu 3,6-Di-O-acetyl-D-glucal (kontinuierlicher Säulenbetrieb)

Eine Lösung von 5,21 g Glucal wird in 50 ml Dimethoxyethan (DME)gelöst, mit 500 ml Vinylacetat versetzt und im diskontinuierlichen Verfahren 20 Std. umgepumpt (12 g Lipase P auf 120 ml Seesand).

Man erhält 7,39 g des gewünschten 3,6-Diacetats (90 %), das nach DC und $^1$H-NMR sauber ist.

5) Regioselektive Monoacetylierung von D-Glucal zu 6-O-Acetyl-D-glucal (kontinuierlicher Säulenbetrieb)

Man bereitet eine Säule aus 20 g Lipase OF (Candida Cylindracaea) und 200 ml Seesand vor. 5 g D-Glucal werden in 50 ml Dimethoxyethan gelöst und zusammen mit 500 ml Vinylacetat über die Säule gegeben. Die erhaltene heterogene Mischung wird nach einmaligem Passieren der Säule (~ 5 h) für weitere 20 Std. im Kreis gepumpt.

Man erhält nach Abdestillieren des Lösemittels im Vakuum und Kieselgelchromatographie 5,07 g (79 %) des gewünschten Monoacetats, das nach DC und $^1$H-NMR sauber ist.

6) Lipase OF - katalysierte Herstellung von Geranyllaurat (kontinuierlicher Säulenbetrieb)

Die Säulenfüllung besteht aus 20 ml Seesand und 2 g Lipase OF. 200 mg Dodecansäure und 154 mg Geraniol werden in 100 ml Hexan gelöst und über die Enzymensäule gegeben (Fluß ~ 10 ml/min). Das Reaktionsgemisch enthält ca. 95 % Produkt und 5 % Edukte (DC) und wird durch Kieselgelchromatographie gereinigt. Man erhält 222 mg Geranyllaurat, das nach DC und $^1$H-NMR einheitlich ist.

Weitere Beispiele sind in Tabelle 1 aufgeführt.

Alle in Tabelle 1 durchgeführten Experimente werden im kontinuierlichen Verfahren und unter Verwendung der Pseudomonas-Lipase durchgeführt.

| Bsp.-Nr. | Verbindungen | Träger | Edukt | Konz. | Träger | Enzym | Vinyl-acetat | Umsatz (4) | Reak.-geschw. (3) | ee Acetat/Alkohol (1) |
|---|---|---|---|---|---|---|---|---|---|---|
| 7 | 1-Phenylethanol (2) | Seesand | 0,5 g | 1 % | 50 ml | 5 g | 50 ml | 45 % | + | >95%/>95% |
| | | Seesand | 50 g | 5 % | 50 ml | 5 g | 1000 ml | 43 % | | >95%/>93% |
| | | Seesand | 500 g | 10 % | 500 ml | 50 g | 5000 ml | 48 % | | >95%/>95% |
| | | Ziegel-mehl | 5 g | 5 % | 50 ml | 5 g | 100 ml | 46 % | | >95%/>95% |
| 8 | 2-Phenylpropanol | Seesand | 0,5 g | 0,1 % | 50 ml | 5 g | 50 ml | 31 % | - | >95%/n.b. |
| 9 | 2-Chlor-1-phenylethanol | Seesand | 0,5 g | 0,2 % | 50 ml | 5 g | 50 ml | 28 % | - | >95%/n.b. |

EP 0 507 278 A2

| Bsp.-Nr. | Verbindung | Träger | Edukt | Konz. | Träger | Enzym | Vinyl-acetat | Umsatz (4) | Reak.-geschw. (3) | ee Acetat/Alkohol (1) |
|---|---|---|---|---|---|---|---|---|---|---|
| 10 | Pantolacton | Seesand | 0,5 g | 0,5 % | 50 ml | 5 g | 100 ml | 20 % | - | >90%/n.b. |
|  |  | Seesand | 10 g | 0,2 % | 500 ml | 50 g | 5000 ml | 58 % (50°C) |  | 14%/85% |
| 11 | 2-Hydroxy-propanaldimethyl-acetal | Seesand | 5 g | 1 % | 50 ml | 5 g | 500 ml | 37 % | + | n.b./n.b. |
| 12 | Allethrolon | Seesand | 0,5 g | 1 % | 50 ml | 5 g | 50 ml | 51 % | + | >95%/n.b. |

| Bsp.-Nr. | Verbindung | Träger | Edukt | Konz. | Träger | Enzym | Vinyl-acetat | Umsatz (4) | Reak.-geschw. (3) | ee Acetat/Alkohol (1) |
|---|---|---|---|---|---|---|---|---|---|---|
| 13 | [2-(6-Acetoxy)-naphthyl]-1-ethanol | Seesand | 14 g | 1 % | 250 ml | 5 g | 1400 ml | 42 % | + | 92%/67% |
| 14 | 1-Octen-3-ol | Seesand | 10 g | 2 % | 50 ml | 5 g | 500 ml | 52 % | - | 85%/84% |
| 15 | 2-(1-Naphthyl-methyl)-propan-1,3-diol | Seesand | 20 g | 2 % | 50 ml | 5 g | 1000 ml | 79 % | + | 60%/- |

EP 0 507 278 A2

Erläuterung zu Tabelle 1:

n.b.:   nicht bestimmt

(1)   Bei Beispiel 7 (1-Phenylethanol) erfolgt eine zusätzliche Bestimmung des ee-Wertes mit HPLC (®Chiralcel OB).

(2)   Bestimmung des ee-Wertes erfolgte per $^1$H-NMR-Shift-Versuche mit Eu(hfc)$_3$ (Acetate) bzw. in gleicher Weise nach der chemischen Acetylierung der Alkohole.

(3)   Die Reaktionsgeschwindigkeiten wurden wie folgt eingeordnet:

+ = schnell d.h. >1g/l/h

- = langsam d.h. <1g/l/h

(4)   Umsatzbestimmung per GC oder $^1$H-NMR.

Die Vorteile der Erfindung liegen in

1. der einfachen Herstellung des immobilisierten Biokatalysators, indem Enzym und Träger miteinander vermischt werden, ohne daß eine vorherige Derivatisierung von Enzym und Träger notwendig ist,
2. der hohen Halbwertszeit des immobilisierten Biokatalysators, da die langen Standzeiten der Katalysatorsäulen arbeits- und kostensparend sind,
3. der Nicht-Auswaschung des Enzyms durch Lösungsmittel, was wiederum eine häufige Wiederverwendbarkeit und lange Standzeiten garantiert und
4. der leichten und kostengünstigen Möglichkeit zur Entsorgung des immobilisierten Biokatalysators, da es sich bei den Trägern um natürliche Materialien handelt.

**Patentansprüche**

1.   Immobilisierter Biokatalysator, aus einem inerten, anorganischen Träger, und einer Hydrolase, die an diesen Träger immobilisiert ist.

2.   Immobilisierter Biokatalysator nach Anspruch 1, dadurch gekennzeichnet, daß der Träger Seesand, Gesteins- oder Ziegelmehl, Glas oder pulverisierte Keramik ist.

3.   Immobilisierter Biokatalysator nach Anspruch 1, dadurch gekennzeichnet, daß die Hydrolase eine Lipase ist.

4.   Immobilisierter Biokatalysator nach Anspruch 1 und 3, dadurch gekennzeichnet, daß als Hydrolyse eine Lipase aus Pseudomonas, Mucor, Candida, Rhizopus, Penicillium oder die Schweinepankreas-Lipase eingesetzt wird.

5.   Immobilisierter Biokatalysator nach Anspruch 4, dadurch gekennzeichnet, daß die Pseudomonas-Lipase P/FP/PS oder die Candida-Lipase verwendet wird.

9

**6.** Immobilisierter Biokatalysator nach Anspruch 1, dadurch gekennzeichnet, daß die Korngröße der Träger maximal 2 mm beträgt.

**7.** Verfahren zur Herstellung eines immobilisierten Biokatalysators, dadurch gekennzeichnet, daß eine Hydrolase mit einem inerten, anorganischen Träger, vorzugsweise aus Seesand, Gesteins- oder Ziegelmehl, Glas oder pulverisierter Keramik gemischt wird und daran immobilisiert ist.

**8.** Verfahren nach Anspruch 7, dadurch gekennzeichnet, daß die Umsetzung im diskontinuierlichen Verfahren erfolgt.

**9.** Verfahren nach Anspruch 7, dadurch gekennzeichnet, daß die Umsetzung im kontinuierlichen Verfahren erfolgt.

**10.** Verfahren nach einem oder mehreren der Ansprüche 7 bis 9, dadurch gekennzeichnet, daß der Träger unbehandelt zur Immobilisierung eingesetzt wird.

**11.** Verwendung des Katalysators nach den Ansprüchen 1 bis 6 oder des nach den Ansprüchen 7 - 10 erhältlichen immobilisierten Biokatalysators zur Estersynthese in einem Säulenreaktor.

**12.** Verwendung des Katalysators nach den Ansprüche 1 bis 6 oder des nach den Ansprüchen 7 - 10 erhältlichen immobilisierten Biokatalysators zur enantio-, diastereo- und regiodifferenzierenden Veresterung durch Acylierung von Alkoholen mit Carbonsäuren und Carbonsäurederivaten.

**13.** Verwendung des immobilisierten Biokatalysators nach Anspruch 12, wobei für die Acylierungsreaktion folgende Alkohole bzw. Carbonsäuren eingesetzt werden:
1-Phenylethanol, 1-Phenylpropanol, 2-Chlor-1-phenylethanol, (+-)-Dihydro-4,4-dimethyl-3-hydroxy-2-(3H)-furanon (Pantolacton), 2-Hydroxypropandimethylacetal, 4-Hydroxy-3-methyl-2-(prop-2-enyl)-cyclopent-2-enon, [2-(6-Acetoxy)naphthyl]-1-ethanol, 1-Octen-3-ol, 2-(1-Naphthylmethyl)-propan-1,3-diol, 1-(4-Isobutylphenyl)-ethanol,Geraniol, Dodecansäure oder 1,5-Anhydro-D-arabinon-hex-1-enitol (Glucal) eingesetzt werden.